(19) Europäisches Patentamt
European Patent Office
Office européen des brevets

(11) Veröffentlichungsnummer: **0 119 546**
**B1**

(12) **EUROPÄISCHE PATENTSCHRIFT**

(45) Veröffentlichungstag der Patentschrift: **30.05.90**

(21) Anmeldenummer: **84102409.4**

(22) Anmeldetag: **07.03.84**

(60) **Teilanmeldung 87108758 eingereicht am 19.06.87.**

(51) Int. Cl.[5]: **C 07 C 403/16,**
C 07 C 49/713, C 07 C 49/743,
C 07 C 49/603, C 07 C 49/647,
C 07 C 45/42, C 07 C 69/145

(54) **Neues Verfahren zur Herstellung von Verbindungen der 4-Oxodamascon-Reihe sowie neue Riechstoffe aus dieser Verbindungsklasse.**

(30) Priorität: **15.03.83 DE 3309169**

(43) Veröffentlichungstag der Anmeldung:
**26.09.84 Patentblatt 84/39**

(45) Bekanntmachung des Hinweises auf die Patenterteilung:
**30.05.90 Patentblatt 90/22**

(84) Benannte Vertragsstaaten:
**CH DE FR GB LI**

(56) Entgegenhaltungen:
**DE-A-2 353 468**

(73) Patentinhaber: **BASF Aktiengesellschaft**
**Carl-Bosch-Strasse 38**
**D-6700 Ludwigshafen (DE)**

(72) Erfinder: **Janitschke, Lothar, Dr.**
**Wormser Gasse 9 a**
**D-6711 Kleinniedesheim (DE)**
Erfinder: **Hoffmann, Werner, Dr.**
**Ringstrasse 11 c**
**D-6708 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 119 546 B1

**Beschreibung**

Es ist aus der DE—OS 2 353 468 bekannt, daß man die Verbindungen der allgemeinen Formel

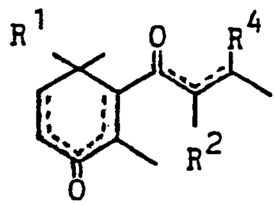

in der $R^1$ bis $R^4$ für Wasserstoff oder niedere Alkylgruppen steht und eine der gestrichelten Linien eine weitere C—C-Bindung bedeutet, wertvolle und interessante Riechstoffe sind. Insbesondere werden hier das als 4-Oxo-damascon bezeichnete 2,6,6-Trimethyl-1-(1-oxo-but-2-en-1-yl)-cyclohex-1-en-3-on (Ia) und seine beiden Hydrierprodukte der Formeln

Ia

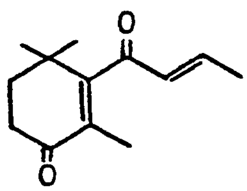

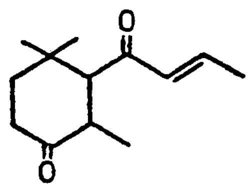

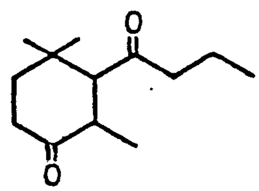

sowie deren Herstellung und Verwendung als Riech- und Geschmackstoffe beschrieben. Für die Verbindung Ia ist ein charakteristischer, süßer, fruchtiger, insbesondere an Birnen und Äpfel erinnernder Geruch angegeben, der sich deutlich von dem fruchtig-minzig, etwas herben Geruch des β-Damascons selbst unterscheidet. Zur Herstellung dieser Verbindungen geht man gemäß der genannten DE—OS jeweils von β-Damascon aus. Aus β-Damascon wird entweder zunächst ein Epoxid in 2,3- oder 3,4-Stellung des Ringes hergestellt, welches durch Säuren in das 4-Oxo-Damascon überführt werden kann, oder aber mit Hilfe von N-Bromsuccinimid das in 3-Stellung des Ringes bromierte Damascon hergestellt, welches mit Chromdioxid in Eisessig/Wasser zu Ia oxidiert werden kann.

Die hier beschriebenen Verfahren befriedigen jedoch technisch noch nicht, da die gewünschten Verbindungen, ausgehend von einem hochveredelten und daher teuren Wertprodukt, dem Damascon, hergestellt werden müssen und zudem auch nur in mäßigen Ausbeuten erhalten werden.

Es war daher die Aufgabe der Erfindung, ein Verfahren zu entwickeln, mit dessen Hilfe man 4-Oxo-damascon und verwandte Verbindungen ausgehend von billigeren Ausgangsstoffen auf einfache und vorteilhafte Weise in guten Ausbeuten herstellen kann.

Gegenstand der Erfindung ist ein Verfahren zur Herstellung der Verbindungen der Formeln Ia und Ib,

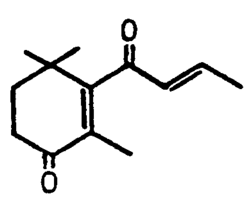

Ia

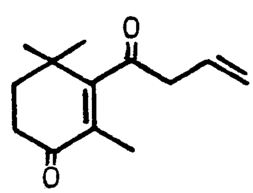

Ib

das dadurch gekennzeichnet ist, daß man

A. das 2,4,4-Trimethyl-cyclohex-2-en-1-on-oxim der Formel IIb

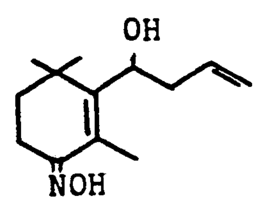

(IIb)

bei Temperaturen von 30 bis 80°C, vorzugsweise bei 40 bis 70°C, mit einer starken wäßrigen Säure behandelt,

B. den erhaltenen Alkohol der Formel IIIb

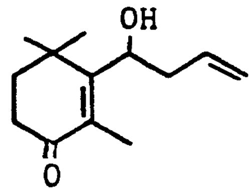

(IIIb)

in an sich bekannter Weise oxydiert und

C. die erhaltene Verbindung Ib gegebenenfalls in an sich bekannter Weise isomerisiert.

Die Behandlung des Oxims der Formel IIb mit starken wäßrigen Säuren bei erhöhter Temperatur wurde bereits in der nicht vorveröffentlichten Patentanmeldung (P 32 31 189.3) zur Herstellung von Megastigma-5,7,9-trien-4-on vorgeschlagen. Es war überraschend, daß man anstelle von dem Megastigmatrienon das neue Keton IIIb erhält, wenn man die Säurebehandlung nicht in dem dort beschriebenen Temperatur-Vorzugsbereich von 90 bis 120, insbesondere 100 bis 115°C, sondern bei Temperaturen von 30 bis 80, vorzugsweise 40 bis 70°C, durchführt.

Der neue Alkohol der Formel IIIb eröffnet nun einen sehr vorteilhaften Weg zu dem begehrten 4-Oxo-β-damascon der Formel Ia, da es einerseits leicht zugänglich ist und andererseits durch Oxydation z.B. mit Chromschwefelsäure in guten Ausbeuten das noch nicht mit physikalischen Daten beschriebene "4-Oxo-β-isodamascon" der Formel Ib bildet, welches durch säurekatalysierte Isomerisierung auf einfache Weise in das gewünschte 4-Oxo-β-damascon überführt werden kann.

Ausgangsverbindung für die Herstellung der Verbindung IIIb ist das entsprechende neue Oxim der allgemeinen Formel IIb

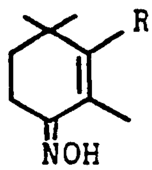

(II)

in der

R für $—CH(OH)—CH_2—CH=CH_2$ (IIb) steht, das seinerseits durch Grignardreaktionen mit der entsprechenden Alkenylgrignardverbindung aus 4-Oximidocyclocitral der Formel IV

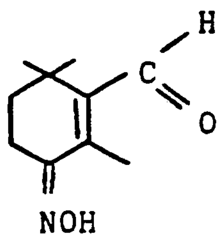

(IV)

in ausgezeichneten Ausbeuten erhalten werden kann.

Gegenstand der Erfindung ist weiterhin die Verbindung der Formel IIIb

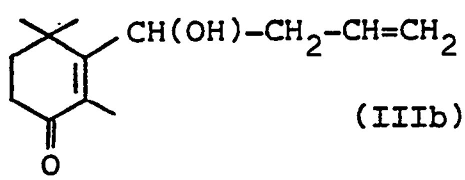

(IIIb)

die als Zwischenprodukt für das erfindungsgemäße Verfahren dient.

Die Verbindungen IIIb, Ib, Ic und Ie sind interessante Riech- und Aromastoffe. Weiterhin können sie als Zwischenprodukte für neue Carotinoide Bedeutung gewinnen. Sie können auf relativ einfache Weise aus recht gut zugänglichen Verbindungen hergestellt werden.

Da IV gemäß dem Verfahren der nicht vorveröffentlichten Patentanmeldung (P 31 37 802.1) auf überraschend einfache Weise und in hoher Ausbeute aus Cyclocitral hergestellt werden kann, und dieses

gemäß der DE—OS 30 27 689 sehr vorteilhaft aus Citral zugänglich ist, ergibt sich somit ein sehr vorteilhaftes Gesamtverfahren zur Herstellung von 4-Oxo-damascon und ähnlichen guten Riech- und Aromastoffen.

Bei der Behandlung von IIb mit starken wäßrigen Säuren bildet sich bei Temperaturen von etwa 30°C bis 80°C, vorzugsweise etwa 40 bis 70°C, IIIb als Hauptprodukt neben etwas Megastigma-5,7,9-trien-4-on. Unterhalb 30°C verlangsamt sich die Umsetzung stark. Als starke wäßrige Säure kommt insbesondere 5 bis 50 gew.%ige Schwefelsäure in Betracht. Auch 1 %ige Schwefelsäure kann noch verwendet werden, jedoch ist es bei so hoher Verdünung der Säure von Vorteil, die Temperatur etwas höher zu wählen. Als stark wäßrige Säuren sind auch die Lösungen bzw. Aufschwämmungen von Phosphorsäure, Chloressigsäure, p-Toluolsulfonsäure oder Suspensionen von stark sauren Kationenaustauschern geeignet. Die Reaktion kann diskontinuierlich, aber auch kontinuierlich durchgeführt werden.

IIIb, das man als "4-Oxo-β-isodamascol" bezeichnen könnte, läßt sich in an sich bekannter Weise, z.B. mit Chromschwefelsäure zu 2,4,4-Trimethyl-3-(but-3-en-1-on-1-yl)-cyclohex-2-en-1-on (Ib) oxidieren. Die Umsetzung läßt sich jedoch auch mit anderen für diese Art von Oxidation geeigneten Reagentien, wie aktivem Braunstein, Pyridiniumchlorochromat, Chromtrioxid-Pyridin-Komplexen oder ähnlichen, in an sich bekannter Weise durchführen. Bezüglich detaillierter Angaben über solche Oxidationen verweisen wir auf das Lehrbuch Jerry March "Advanced Organic Chemistry", 2nd. Ed. 1977, S. 1082, sowie dort zitierte Literatur.

Durch Isomerisierung der Doppelbindung in *Ib* in an sich bekannter Weise, beispielsweise unter saurer Katalyse etwa mit p-Toluolsulfonsäure, erhält man hieraus *Ia*, das 2,4,4-Trimethyl-3-(but-2-en-1-onyl)-cyclohex-2-en-1-on (Ia; 4-Oxo-β-damascon). *Ib* kann jedoch auch bereits bei seiner Destillation, möglicherweise in Gegenwart von Spuren einer Säure, zu *Ia* isomerisiert werden (vgl. z.B. Japan. Patent 75 69 047 (08.06.1975)).

Mit Hilfe des erfindungsgemäßen Verfahrens ist es möglich, das begehrte 4-Oxo-β-damascon *Ia* ohne Verwendung von N-Bromsuccinimid ausgehend von dem einfachen und in hohen Ausbeuten erhältlichen *IIb* in nur 3 einfachen Stufen zu erhalten. *IIb* stellt ein günstigeres Ausgangsmaterial als das gemäß dem Verfahren der DOS 2 353 468 benutzte Damascon dar.

Die Zwischenprodukte IIIb und Ib sind außerdem interessante Riech- und Aromatstoffe. Weiterhin können sie als Zwischenprodukte für neue Carotinoide Bedeutung gewinnen. Sie können auf relativ einfache Weise aus recht gut zugänglichen Verbindungen hergestellt werden.

Beispiel 1

A. Herstellung von 2,4,4-Trimethyl-3-(1-hydroxy-but-3-en-1-yl)-cyclohex-2-en-1-on-oxim (IIb)

Zu 97,2 g (4 mol) Magnesium und 100 ml absolutem Tetrahydrofuran (THF) wurden 306 g (4 mol) Allylchlorid in 2 l abs. THF getropft. (Die Grignardreaktion wurde durch eine Spur Iod und 1 ml reines Allylchlorid gestartet.) Nach vollendeter Zugabe wurde 1 h bei 35 bis 40°C nachgerührt.

Zu der erhaltenen Lösung von Allylmagnesiumchlorid wurde bei Raumtemperatur eine Lösung von 181 g (1 mol) 1-Formyl-2,6,6-trimethyl-3-oximido-cyclohex-1-en (II) in 200 ml THF getropft. Anschließend wurde 1 h bei Raumtemperatur nachgerührt, dann unterhalb von 10°C 400 ml $H_2O$ zugetropft, 15 min nachgerührt, der gebildete Niederschlag abgesaugt und der Filterrückstand 3 mal mit je 250 ml $CHCl_3$ nachgewaschen. Die vereinigten Filtrate wurden bei 20 mbar eingeengt und der kristalline Rückstand (207 g; Rohausbeute 93%) aus 70 ml Cyclohexan umkristallisiert.

Man erhielt 190 g IIIa vom Schmelzpunkt F = 106—111°C. Das entspricht einer Ausbeute von 85% der Theorie.

B. Herstellung von 2,4,4-Trimethyl-3-(1-hydroxy-but-3-en-1-yl)-cyclohex-2-en-1-on (*IIIb*)

193 g (0,86 mol) eines gemäß A hergestellten 2,4,4-Trimethyl-3-(1-hydroxy-but-3-en-1-yl)-cyclohex-2-en-1-on-oxims (IIb) wurden zusammen mit 1900 ml 30 %iger wäßriger $H_2SO_4$ 6 h bei 55°C gerührt. Danach wurde das Reaktionsgemisch auf RT abgekühlt und mehrmals mit Methyl-t-butylether extrahiert. Die vereinigten Extrakte wurden mit Natriumsulfat getrocknet, das Lösungsmittel bei 20 mbar abdestilliert.

Man erhielt 164 g rohes 2,4,4-Trimethyl-3-(1-hydroxy-but-3-en-1-yl)-cyclohex-2-en-1-on (*IIIb*; Ausbeute: 91,2% d. Th.). Nach Rohdestillation ($Kp_{0,5-0,1\ mbr}$ = 62—142°C) erhielt man 148 g eines gemäß Dünnschichtchromatographie leicht verunreinigten Produktes (Ausbeute: 82,3% d. Th.). Ein dünnschichtchromatographisch reines Produkt läßt sich durch Umkristallisation aus 445 ml Cyclohexan gewinnen (94 g reines Produkt, Ausbeute: 52,2% und 37,3 g eingeengtes Filtrat, leicht verunreinigtes Produkt, Ausbeute: 20,8% d. Th.).

IR (KBr-Preßling): 3 434 $cm^{-1}$(OH); 3 078 $cm^{-1}$ (=C—H); 1 656 $cm^{-1}$ (unges. C=O); 1 611 $cm^{-1}$, 1 595 $cm^{-1}$(C=C).

$^1$H—NMR ($CDCl_3$/200 MHz) δ: 5,91 ppm, m, 1H, CH—3'; 5,29 ppm, d, 5,28 ppm, d, $CH_2$—4'; 4,55 ppm, d, d (J1~3Hz, J2 10 Hz), 1H, CH—1'; 2,49 ppm, t (J~7Hz), 2H, $CH_2$—6; 2,05 ppm, sbr., 1H, OH; 1,97 ppm, S, 3H, $CH_3$ an C—2; 1,83 ppm, t (J~7Hz), 2H, $CH_2$—5; 1,33 und 1,23 ppm, je 1S, je 3H$CH_3$ an C—4.

$^{13}$C—NMR ($CDCl_3$/90,52 MHz) δ: 200,10 ppm, C—1; 163,57 ppm, C—3; 134,94 ppm, C—3'; 131,77 ppm, C—2; 118,14 ppm, C—4'; 70,71 ppm, C—1'; 40,33 ppm, C—2'; 37,86 ppm, C—5; 36,01 ppm, C—4; 34,10 ppm, C—6; 27,28 ppm und 26,14 ppm, $CH_3$ an C—4; 12,54 ppm, $CH_3$ an C—2 Geruch: leicht blumig, brenzlig.

C. Herstellung von 2,4,4-Trimethyl-3-(but-3-en-1-on-1-yl)-cyclohex-2-en-1-on (Ib)

Zu einer Lösung von 67 g (0,32 mol) eines gemäß 1B hergestellten 2,4,4-Trimethyl-3-(1-hydroxy-but-3-en-1-yl)-cyclo-hex-2-en-1-ons (IIIb) in 670 ml Diethylether wurde bei 20 bis 25°C eine Lösung von 57,8 g (0,19 mol) $Na_2Cr_2O_7 . 2H_2O$ in 312 ml Wasser und 42 ml konzentrierte Schwefelsäure getropft. Es wurde 4 h bei 20 bis 25°C nachgerührt und die organische Phase abgetrennt. Die wäßrige Phase wurde mit Diethylether (Ether) extrahiert. Die vereinigten organischen Phasen wurden mit Wasser und mit gesättigter Natriumhydrogencarbonatlösung gewaschen sowie mit Natriumsulfat getrocknet. Das Lösungsmittel wurde bei 20 mbar abdestilliert. Man erhielt 60 g eines nach Dünnschichtchromatographie (DC) leicht verunreinigten 2,4,4-Trimethyl-3-(but-3-en-1-on-1-yl)cyclohex-2-en-1-ons (Ausbeute: 90,9% d. Th.). Durch Kristallisation aus 500 ml Cyclohexan wurden 37 g reines Produkt (Ausbeute: 56% d. Th.) gewonnen. Durch Abdestillieren des Lösungsmittels erhielt man aus der Mutterlauge weitere 21 g leicht verunreinigtes Produkt (31,8% d. Th.).

Fp: 52—61°C

Kp: 0,5 mbar = 116—120°C

IR (KBr-Preßling):

$^1H$—NMR ($CDCl_3$/100 MHz) δ: 6,21—5,72 ppm, "m", 1H, CH—3'; 5,28—5,02 ppm, "m", 2H, $CH_2$—4'; 3,37 ppm, t, d (J1~2Hz, J2~7Hz), 2H, $CH_2$—2'; 2,54 ppm, t ( ~7Hz), 2H, $CH_2$—6, 1,92 ppm, t (J~7Hz), 2H, $CH_2$—5; 1,65 ppm, S, 3H, $CH_3$ an C—2; 1,23 ppm, S, 6H, $CH_3$ an C—4.

$^{13}C$—NMR ($CDCl_3$/90,52 MHz) δ: 205,47 ppm, C—1'; 198,45 ppm, C—1; 162,44 ppm, C—3; 129,25 ppm, C—3'; 127,95 ppm, C—2; 119,37 ppm, C—4'; 48,95 ppm, C—2'; 38,03 ppm, C—5; 34,44 ppm, C—4; 34,12 ppm, C—6; 27,03 ppm, $CH_3$ an C—4 12,77 ppm, $CH_3$ an C—13.

Geruch: apfelartig.

D. Herstellung von 2,4,4-Trimethyl-3-(but-2-en-1-on-1-yl)-cyclohex-2-en-1-on (*Ia*)

153 g (0,74 mol) 2,4,4-Trimethyl-3-(but-3-en-1-on-1-yl)-cyclohex-2-en-1-on (Ib) wurden zusammen mit 135 ml Essigester und 3 g p-Toluolsulfonsäure 2 h unter Rückflüßbedingungen gerührt. Nach dem Abkühlen wurde die Lösung mit 1000 ml Ether versetzt, mit Wasser und mit ges. Natriumhydrogencarbonatlösung gewaschen sowie mit Natriumsulfat getrocknet. Die Lösungsmittel wurden bei ca. 20 mbar abdestilliert. Man erhielt 153 g eines gemäß DC nur leicht verunreinigten 2,4,4-Trimethyl-3-(but-2-en-1-on-1-yl)-cyclohex-2-en-1-on (*Ia*; Ausbeute: quantitativ). Durch Umkristallisation aus 500 ml Cyclohexan wurden 132 g eines gemäß DC reinen Produkts (Ausbeute: 86,8% d.Th.) und nach Einengen der Mutterlauge weitere 21 g eines etwas verunreinigten Produkts (Ausbeute: 13,2% d. Th.) erhalten.

Geruch: süß, fruchtig, an Äpfel und Birnen erinnernd.

## Beispiel 2

Jeweils die in der folgenden Tabelle angegebene Menge an 2,4,4-Trimethyl-3-(1-hydroxy-but-3-en-1-yl)-cyclohex-2-en-1-on-oxim (IIb) wurden zusammen mit der aus der Tabelle ersichtlichen wäßrigen Säure die dort angegebene Zeit unter Rühren auf die dort angegebene Temperatur erhitzt. Nach dem Abkühlen wurde das Reaktionsgemisch mehrmals mit Ether extrahiert, die vereinten Extrakte mit ges. $NaHCO_3$-Lösung gewaschen und mit Natriumsulfat getrocknet. Das Lösungsmittel wurde bei ca. 20 mbar abdestilliert und in dem so erhaltenen Rohprodukt mittels H—NMR-Spectroskopie oder Gaschromatographie analysiert.

Tabelle

| Beispiel | IIb [g/mmol] | wäßrige Säure [ml oder g] | Temperatur [°C] | Zeit [h] | Rohprodukt [g] | enthaltend |
|---|---|---|---|---|---|---|
| 2a | 11,05/49,1 | 110 g 30%ige $H_2SO_4$ | 80 | 6 | 8,9 | ca.98 % IIIb |
| 2b | 11,15/49,6 | 110 g 10%ige $H_2SO_4$ | 60 | 3 | 10,7 | ca.97 % IIIb |
| 2c | 11,5 /51 | 110 g 30%ige $H_3PO_4$ | 60 | 3 | 11 | ca.74 % IIIb; 26 % IIb |
| 2d | 11,5 /51 | 110 g 30%ige Chloressigsäure | 60 | 3 | 10 | ca.36 % IIIb; ca.64 % IIb |
| 2e | 11 /48,8 | 110 ml 30 %ige p-Toluolsulfonsäure | 60 | 3 | 11 | ca.60 % IIIb; 38 % IIb |
| 2f | 11,5 /51 | 50 ml Ionenaustauscher A 15$H^+$+50 ml $H_2O$ | 60 | 3 | 6 | ca.99,6 % IIIb |

**Patentansprüche**

1. Verfahren zur Herstellung der Verbindungen der Formeln Ia und Ib,

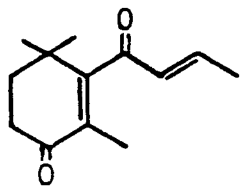

Ia

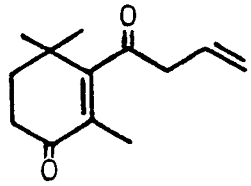

Ib

dadurch gekennzeichnet, daß man

A. das 2,4,4-Trimethyl-cyclohex-2-en-1-on-oxim der Formel IIb

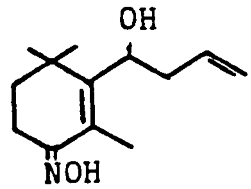

(IIb)

bei Temperaturen von 30 bis 80°C, vorzugsweise bei 40 bis 70°C, mit einer starken wäßrigen Säure behandelt,

B. den erhaltenen neuen Alkohol der Formel IIIb

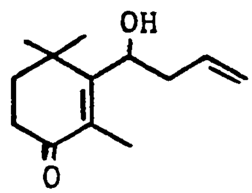

(IIIb)

in an sich bekannter Weise oxydiert und

C. die erhaltene Verbindung Ib gegebenenfalls in an sich bekannter Weise isomerisiert.

2. 2,4,4-Trimethyl-3-(1-hydroxy-but-3-en-1-yl)-cyclohex-2-en-1-on (IIIb).

**Revendications**

1. Procédé de préparation de composés de formules Ia et Ib,

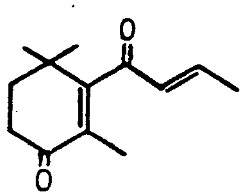

Ia

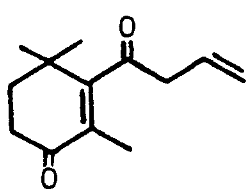

Ib

caractérisé par le fait que

A. on traite le 2,4,4-triméthyl-cyclohex-2-en-1-on-oxime de formule IIb

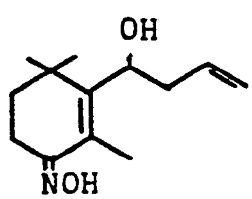

(IIb)

à des températures de 30 à 80°C, de préférence entre 40 et 70°C, avec un acide aqueux fort,

B. on oxyde, de manière connue en soi, le nouvel alcool obtenu, de formule IIIb

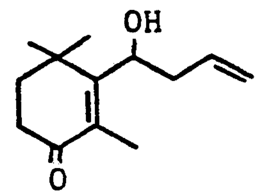

(IIIb)

C. on isomérise éventuellement, de manière connue en soi, le composé Ib obtenu.

2. 2,4,4-triméthyl-3-(1-hydroxy-but-3-en-1-yl)-cyclohex-2-en-1-one (IIIb).

**Claims**

1. A process for the preparation of the compounds of the formulae Ia and Ib

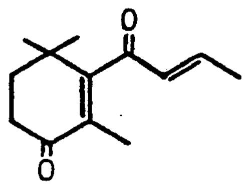

Ia

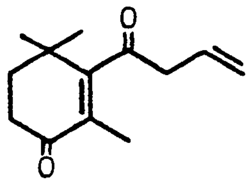

Ib

wherein

A. 2,4,4-trimethylcyclohex-2-en-1-one oxime of the formula IIb

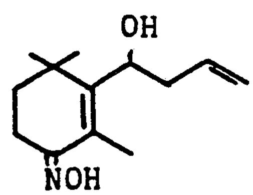

(IIb)

is treated with a strong aqueous acid at from 30 to 80°C, preferably from 40 to 70°C,

B. the resulting novel alcohol of the formula IIIb

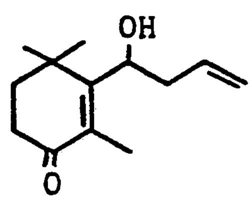

(IIIb)

is oxidized in a conventional manner, and

C. if desired, the resulting compound Ib is isomerized in a conventional manner.

2. 2,4,4-Trimethyl-3-(1-hydroxybut-3-en-1-yl)-cyclohex-2-en-1-one (IIIb).